# EUROPEAN PATENT SPECIFICATION

(11) **EP 0 646 388 B1**
(45) Date of publication and mention of the grant of the patent: **17.02.1999**
(21) Application number: 94112849.8
(22) Date of filing: 17.08.1994
(51) Int. Cl.: A61N 1/05

(54) **Electrode device**
Elektrodenvorrichtung
Dispositif d'électrodes

(30) Priority: 24.09.1993 SE 9303120
(43) Date of publication of application: 05.04.1995
(73) Proprietor: Pacesetter AB, 175 84 Järfälla (SE)
(72) Inventor: Högnelid, Kurt, S-137 38 Västerhaninge (SE); Obel, Martin, S-182 33 Danderyd (SE); Thornander, Hans, F-77760 Recloses (FR)
(74) Representative: Lettström, Richard Wilhelm

(56) References cited:
- EP-A- 0 534 401
- US-A- 3 915 174
- US-A- 4 258 724

## Description

The present invention relates to an electrode device intended to be at least partially implanted into living tissue, comprising an electrically insulating sheath, an electrical conductor arranged in the insulating sheath, an electrode arranged on the insulating sheath for carrying electrical signals between the living tissue and the electrical conductor and a contact arranged at a proximal end of the insulating sheath for carrying electrical signals between the electrical conductor and a medical apparatus.

One such electrode device is described in US-A-3,915,174. The known electrode device can be implanted into a heart either temporarily or permanently in order to stimulate the heart and sense electrical heart signals. The electrode device can be connected either to an extracorporeal apparatus or to an implantable apparatus, such as a pacemaker.

The utilization of temporary electrode devices for sensing electrical signals in body tissue is very useful in conjunction with e.g. diagnosis of illnesses and in determinations of the most appropriate treatment for a patient. One such determination could entail e.g. an investigation of whether the patient should be provided with an implantable defibrillator.

For reception of usable values in the monitoring of electrical signals in living tissue, the electrode device must be connected to the tissue in a reliable manner. There are several different known ways to attach an electrode device to tissue. Basically either an active means or a passive means is used. When the electrode device is attached to the tissue it will, after only a relatively short period of time, become embedded in the tissue. As a result, the electrode device cannot readily be removed once the investigation has been completed. Since the electrode device becomes firmly embedded in tissue, the tissue could be damaged when the electrode device is removed. Explanting a temporary electrode device also requires surgery, as a rule, thereby increasing the risk to the patient. One common recourse for minimizing trauma to tissue and risks to the patient is simply to leave the electrode device inside the patient. However, this solution is not without risk either, since the electrode device, if left behind in the body, could irritate and even damage other tissue.

In the case of pure mechanical implants, which are only needed temporarily in the body of a patient, resorbable materials are now being used to a large degree. The resorbable material gradually dissolves in the body, ultimately disappearing completely, and is usually replaced by living tissue. But the electrical conductor is a problem when electrical signals have to be sensed. No resorbable electrical conductors are currently known.

In a previously submitted European patent application, number 94103706.1, published as EP-A-0 621054 on 26/10/94, an electrode device is described, in which metallic conductors are replaced with non-toxic, liquid conductors in order to reduce the risk of conductor fracture and short-circuits between conductors. When electrical signals from tissue are to be sensed, the liquid conductor can be made of an electrolyte. In principle, the electrolyte can consist of sodium chloride and water, making it completely harmless to tissue.

One object of the invention is to achieve an electrode device, according to the preamble of claim 1, which is suitable as a temporary implantable electrode device for living tissue and which solves the aforesaid problems.

One such electrode device is achieved in accordance with the invention in that the insulating sheath is made of a resorbable material, and in that the electrical conductor is made of a non-toxic, liquid conductor, preferably an electrolyte.

Accordingly, the insulating sheath will ultimately dissolve completely and the liquid conductor will mix with body fluids without causing any damage. The only part remaining in the body will be the electrode itself.

If the electrode is devised with a small contact area to the tissue, e.g. one or a few mm², it does not have to be embedded into the tissue and can be allowed to, for instance, follow the circulating blood when the sheath dissolves.

The electrode is advantageously arranged at a distal end of the insulating sheath, since this facilitates the implantation procedure. It can in this instance be embedded into the tissue during implantation and therefore poses no risk to the tissue.

It is also advantageous if the electrode is devised as a membrane made of an ion-transporting material.

No metallic material would then remain behind in the body, just a thin membrane embedded in the tissue. However, in some cases it may still be preferable to employ electrodes devised of non-toxic, biocompatible metal or carbon-based material.

A refinement of the electrode device is achieved in accordance with the invention in that at least one further electrical conductor is arranged in the insulating sheath, electrically insulated from the first electrical conductor, the further electrical conductor consisting of a non-toxic, liquid conductor, preferably an electrolyte, and in that at least one further electrode is arranged on the insulating sheath to carry electrical signals between the living tissue and the further electrical conductor.

By increasing the number of liquid conductors and electrodes, one and the same electrode device could sense a plurality of tissue parts simultaneously. For example, a plurality of measurement points around or in the heart could be sensed simultaneously. Likewise, several locations along some muscle or a nerve-path may be sensed for electrical signals.

In this context, it is advantageous if the further electrode is devised as a membrane made of an ion-transporting material. As for the electrode, there may be cases in which it is preferable to devise the further electrode of a non-toxic, biocompatible metal or carbon-based material.

Likewise, if the further electrode is devised with a small area, e.g. one or a few mm², it does not have to be affixed to tissue but can follow the circulating blood when the sheath dissolves.

A refinement of the electrode device is achieved in accordance with the invention in that the ion-transporting material is also resorbable.

Referring to the figures, three embodiments of the electrode device according to the invention will be described in greater detail below, wherein:
- FIG. 1: shows a first use of the electrode device according to the invention;
- FIG. 2: shows a first embodiment of the electrode device;
- FIG. 3: shows a second use of the electrode device according to the invention;
- FIG. 4: shows a second embodiment of the electrode device; and
- FIG. 5: shows a third embodiment of the electrode device.

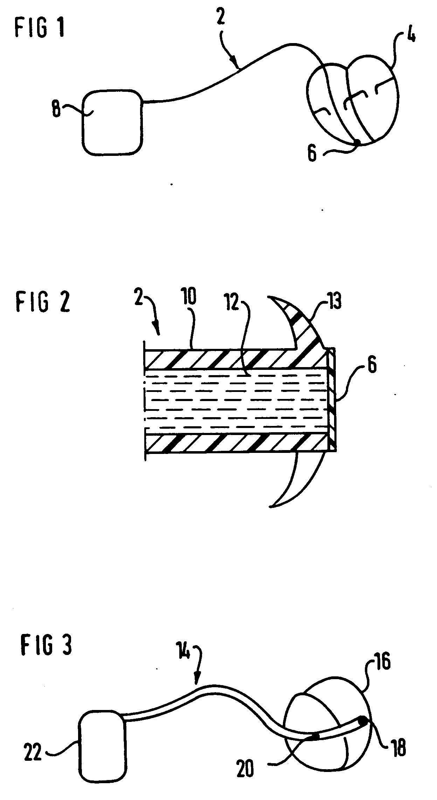

The electrode device 2 in FIG. 1 has been intravenously introduced into a heart 4 and connected to the heart 4 by a tip electrode 6 for sensing electrical signals in the heart 4. At its other end, the electrode device 2 is connected to a monitor 8 which acquires and stores the sensed heart signals. The monitor 8 can either be extracorporeal or implantable.

In FIG. 2 is shown the anterior part of the electrode device 2 in cross-section in order to illustrate the design of the electrode device 2. The tip electrode 6 consists of a thin membrane, made of an ion-transporting material, for detecting signals from heart tissue. An insulating sheath 10 encloses the electrode lead 12 which is made of an electrolyte. In order to affix the tip electrode 6 to the heart tissue, the insulating sheath 10 has been provided with a pair of barb-like projections 13 which are affixed to the heart tissue at implantation. The insulating sheath 10 and the projections 13 are made of a resorbable material. This means that the sheath 10 and the projections 13 will dissolve after a time, and the electrolyte 12 will mix with other body fluids. If the ion-transporting material is also made of a resorbable material, none of the implanted material will remain in the body after a time.

In FIG. 3 is shown another electrode device 14 which is connected to the exterior of a heart 16 by a first electrode 18 and a second electrode 20 for sensing electrical signals in the heart 16. The electrode device 14 is connected to a monitor 22 which detects and stores the sensed electrical signals in the same way as the monitor 8 in FIG. 1.

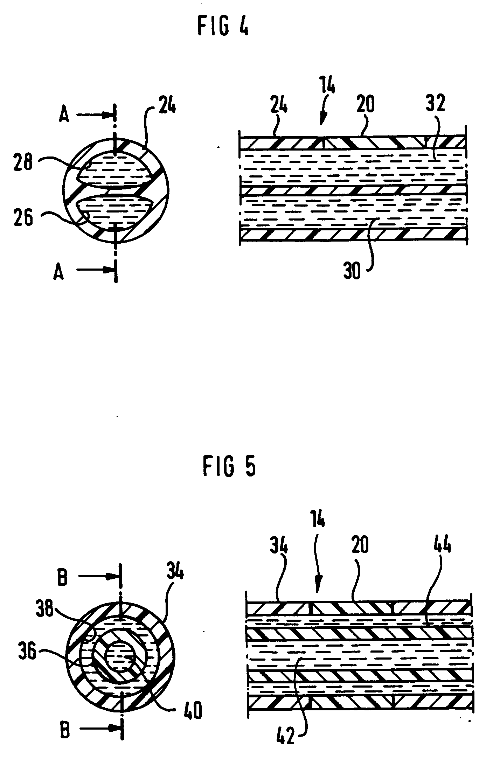

A first version of the electrode device 14 is shown in FIG. 4. An insulating sheath 24 divides the electrode device 14 into a first channel 26 and a second channel 28. The first channel 26 is filled with a first electrolyte 30, and the second channel 28 is filled with a second electrolyte 32.

The first electrode 18 can be formed as the electrode 6 in Fig. 2., i.e. having a thin membrane of a ion-transporting material, which is connected to the first electrolyte 30 in the first channel 26. The second electrode 20 is also devised as a thin membrane made of an ion-transporting material connected to the second channel 28 and, accordingly, to the second electrolyte 32 in order to detect signals from heart tissue and carry them to the monitor 22.

Another version of the electrode device 14 is shown in FIG. 5 in which an insulating sheath 34 encloses an insulating tube 36. This creates a space 38 between the insulating sheath 34 and the insulating tube 36. The insulating tube 36 has a through channel 40. The channel 40 is filled with a first electrolyte 42, and the space 38 between the insulating sheath 34 and the insulating tube 36 is filled with a second electrolyte 44.

In this instance, the second electrode 20 is devised as a thin, annular membrane made of an ion-transporting material which is in contact with the second electrolyte 44 in order to carry electrical signals from the heart 16 to the monitor 22. If the membrane is devised so it only has a very small area, a few mm², it does not even need to be resorbable.

The invention is not limited to the above-described embodiments but can, for example, contain an optional number of electrolyte conductors for sensing at different tissue points in a body. Combinations of the described embodiments are possible.

The electrolyte can be replaced with any non-toxic, liquid conductor. The electrodes can also be made of non-toxic, biocompatible metallic or carbon-based materials.

Whether the monitor is implantable or not, it can contain a plurality of measurement functions contributing information on the patient's state of health. For example, blood pressure and pH are useful parameters in conjunction with recordings of the heart's electrical signal, improving the physician's ability to make a correct diagnosis. In principle, the monitor can be equipped to measure all the variables and parameters of interest to the physician. The recording sensors and signal transmitters affixed to tissue can suitably be made of a resorbable material, whereas the ones, not affixed to tissue and which are easily removed without risk to the patient, can be devised as conventional measurement sensors.

Although the disclosed embodiments only relate to measuring electrical heart signals, the same or similar electrode devices can be used for sensing electrical signals from other living tissue, such as muscles and nerves.

The electrode device may also be employed for temporary electrical stimulation of living tissue, such as muscle, heart and nerves. In this case, a stimulation pulse generator replaces or supplements the monitor.

## Claims

1. Electrode device (2; 14) intended to be at least partially implanted into living tissue, comprising an electrically insulating sheath (10; 24; 34), an electrical conductor (12; 30, 32; 42, 44) arranged in the insulating sheath (10; 24; 34), an electrode (6; 18, 20) arranged on the insulating sheath (10; 24; 34) for carrying electrical signals between the living tissue and the electrical conductor (12; 30, 32; 42, 44) and a contact arranged at a proximal end of the insulating sheath (10; 24; 34) for carrying electrical signals between the electrical conductor (12; 30, 32; 42, 44) and a medical apparatus (8; 22), **characterized in** that the insulating sheath (10; 24; 34) is made of a resorbable material, and in that the electrical conductor (12; 30, 32; 42, 44) consists of a non-toxic, liquid conductor, preferably an electrolyte.

2. Electrode device according claim 1, **characterized in** that the electrode (6; 18, 20) has a contact area to the tissue which is one or a few mm².

3. Electrode device according to claim 1 or 2, **characterized in** that the electrode (6; 18) is arranged at a distal end of the insulating sheath (10; 24; 34).

4. Electrode device according to any of the preceding claims, **characterized in** that the electrode (6; 18, 20) is devised as a membrane made of an ion-transporting material.

5. Electrode device according to any of the claims 1-3, **characterized in** that the electrode (6; 18) is devised of a non-toxic, biocompatible metal or carbon-based material.

6. Electrode device according to any of the preceding claims, **characterized in** that at least one further electrical conductor (32; 44) is arranged in the insulating sheath (24; 34), electrically insulated from the first electrical conductor (30; 42), the further electrical conductor (32; 44) consisting of a non-toxic, liquid conductor, preferably an electrolyte, and that at least one further electrode (20) is arranged on the insulating sheath (24; 34) to carry electrical signals between the living tissue and the further electrical conductor (32; 44).

7. Electrode device according to claim 6, **characterized in** that the further electrode (20) is devised as a membrane made of an ion-transporting material.

8. Electrode device according to claim 6, **characterized in** that the further electrode (20) is devised of a non-toxic, biocompatible metal or carbon-based material.

9. Electrode device according to claim 4 or 7, **characterized in** that the ion-transporting material is also resorbable.

## Patentansprüche

1. Elektrodenvorrichtung (2; 14), die dazu vorgesehen ist, zumindest teilweise in lebendes Gewebe implantiert zu werden, mit einer elektrisch isolierenden Hülle (10; 24; 34), einem in der isolierenden Hülle (10; 24; 34) angeordneten elektrischen Leiter (12; 30, 32; 42, 44), einer auf der isolierenden Hülle (10; 24; 34) angeordneten Elektrode (6; 18, 20) zum Überführen elektrischer Signale zwischen dem lebenden Gewebe und dem elektrischen Leiter (12; 30, 32; 42, 44) und einem am proximalen Ende der isolierenden Hülle (10; 24; 34) angeordneten Kontakt zum Überführen elektrischer Signale zwischen dem elektrischen Leiter (12; 30, 32; 42, 44) und einem medizinischen Gerät (8; 22), **dadurch gekennzeichnet,** daß die isolierende Hülle (10; 24; 34) aus einem resorbierbaren Material gebildet wird und daß der elektrischen Leiter (12; 30, 32; 42, 44) aus einem nichttoxischen, flüssigen Leiter, vorzugsweise einem Elektrolyten besteht.

2. Elektrodenvorrichtung nach Anspruch 1, **dadurch gekennzeichnet,** daß die Elektrode (6; 18, 20) zum Gewebe eine Kontaktfläche hat, die einen oder einige mm² groß ist.

3. Elektrodenvorrichtung nach Anspruch 1 oder 2, **dadurch gekennzeichnet,** daß die Elektroden (6; 18) am distalen Ende der isolierenden Hülle (10; 24; 34) angeordnet ist.

4. Elektrodenvorrichtung nach einem der vorhergehenden Ansprüche, **dadurch gekennzeichnet,** daß die Elektrode (6; 18, 20) als eine aus einem Ionen transportierenden Material bestehende Membran ausgebildet ist.

5. Elektrodenvorrichtung nach einem der Ansprüche 1-3, **dadurch gekennzeichnet,** daß die Elektrode (6; 18) aus einem nichttoxischen, biokompatiblen Metall oder kohlenstoffbasierten Material besteht.

6. Elektrodenvorrichtung nach einem der vorhergehenden Ansprüche, **dadurch gekennzeichnet,** daß mindestens ein weiterer elektrischer Leiter (32; 44) in der isolierenden Hülle ( 24; 34) angeordnet ist, isoliert von dem ersten elektrischen Leiter (30; 42), wobei der weitere elektrische Leiter (32; 44) aus einem nichttoxischen, flüssigen Leiter, vorzugsweise einem Elektrolyten besteht, und daß zumindest eine weitere Elektrode (20) auf der isolierenden Hülle (24; 34) angeordnet ist, um elektrische Signale zwischen dem lebenden Gewebe und dem weiteren elektrischen Leiter (32; 44) zu übertragen.

7. Elektrodenvorrichtung nach Anspruch 6, **dadurch gekennzeichnet,** daß die weitere Elektrode (20) als eine aus einem Ionen transportierenden Material bestehende Membran ausgebildet ist.

8. Elektrodenvorrichtung nach Anspruch 6, **dadurch gekennzeichnet,** daß die weitere Elektrode (20) aus einem nichttoxischen, biokompatiblen Metall oder kohlenstoffbasierten Material besteht.

9. Elektrodenvorrichtung nach Anspruch 4 oder 7, **dadurch gekennzeichnet,** daß das Ionen transportierende Material auch resorbierbar ist.

## Revendications

1. Dispositif (2;14) à électrode, destiné à être implanté au moins partiellement dans du tissu vivant, comprenant une gaine (10;24;34) isolante du point de vue électrique, un conducteur (12;30,32;42,44) électrique disposé dans la gaine (10;24;34) isolante, une électrode (6;18,20) disposée sur la gaine (10;24;34) isolante et destinée à faire passer des signaux électriques entre le tissu vivant et le conducteur (12;30,32;42,44) électrique et un contact disposé à l'extrémité proximale de la gaine (10;24;34) isolante et destiné à faire passer des signaux électriques entre le conducteur (12;30,32;42,44) électrique et un appareil (8;22) médical, caractérisé en ce que la gaine (10;24;34) isolante est en un matériau résorbable et en ce que le conducteur (12;30,32;42,44) électrique consiste en un conducteur liquide et non toxique, qui, de préférence, est un électrolyte.

2. Dispositif à électrode suivant la revendication 1, caractérisé en ce que l'électrode (6;18;20) a une surface de contact avec le tissu qui est de un ou de quelques mm².

3. Dispositif à électrode suivant la revendication 1 ou 2, caractérisé en ce que l'électrode (6; 18) est disposée à l'extrémité distale de la gaine (10;24;34).

4. Dispositif à électrode suivant l'une quelconque des revendications précédentes caractérisé en ce que l'électrode (6;18;20) est constituée sous la forme d'une menbrane en un matériau de transport des ions.

5. Dispositif à électrode suivant l'une quelconque des revendications 1 à 3, caractérisé en ce que l'électrode (6;18) est constituée sous la forme d'un métal ou d'un matériau à base de carbone bio-compatibles et non-toxiques.

6. Dispositif à électrode suivant l''une quelconque des revendications précédentes caractérisé en ce qu'au moins un autre conducteur (32;44) électrique est disposé dans la gaine (24;34) isolante en étant isolé électriquement du premier conducteur (30;42) électrique, l'autre conducteur (32,44) électrique consistant en un conducteur liquide et non toxique, qui est, de préférence, un électrolyte et en ce qu'au moins une autre électrode (20) est disposée sur la gaine (24;34) isolante pour faire passer des signaux électriques entre le tissu vivant et l'autre conducteur (32;44) électrique.

7. Dispositif à électrode suivant la revendication 6, caractérisé en ce que l'autre électrode (20) est constituée sous la forme d'une membrane en un matériau de transport des ions.

8. Dispositif à électrode suivant la revendication 6, caractérisé en ce que l'électrode (20) est constituée sous la forme d'un métal ou d'un matériau à base de carbone bio-compatibles et non-toxiques.

9. Dispositif à électrode suivant la revendication 4 ou 7, caractérisé en ce que le matériau de transport des ions est également résorbable.
